# EUROPÄISCHE PATENTSCHRIFT

(11) **EP 4 015 589 B1**
(45) Veröffentlichungstag und Bekanntmachung des Hinweises auf die Patenterteilung: **09.08.2023**
(21) Anmeldenummer: 21213139.5
(22) Anmeldetag: 08.12.2021
(51) Int. Cl.: C09D 11/08, C09D 7/45, C09D 193/00, C08K 5/1545, C08K 5/00, C09D 5/02, C09D 17/00, C09D 7/63, C09D 7/61

(54) **VERWENDUNG VON RHAMNOLIPIDEN UND/ODER SOPHOROLIPIDEN IN BESCHICHTUNGSZUSAMMENSETZUNGEN ZUR ERHALTUNG EINES HOMOGENEN FARBBILDES DES TROCKENEN BESCHICHTUNGSFILMS UND/ODER ZUR ERHÖHUNG DER ARBEITSSICHERHEIT BEI DER HERSTELLUNG VON BESCHICHTUNGSZUSAMMENSETZUNGEN**
USE OF RHAMNOLIPIDS AND / OR SOPHOROLIPIDS IN COATING COMPOSITIONS FOR MAINTAINING A HOMOGENEOUS COLOUR IMAGE OF THE DRY COATING FILM AND / OR FOR INCREASING WORK SAFETY IN THE PRODUCTION OF COATING COMPOSITIONS
UTILISATION DE RHAMNOLIPIDES ET/OU DE SOPHOROLIPIDES DANS LES COMPOSITIONS DE REVÊTEMENT POUR MAINTENIR UNE IMAGE COULEUR HOMOGÈNE DES FILMS DE REVÊTEMENT SECS ET/OU POUR RENFORCER LA SÉCURITÉ DU TRAVAIL LORS DE LA FABRICATION DE COMPOSITIONS DE REVÊTEMENT

(30) Priorität: 15.12.2020 EP 20214083
(43) Veröffentlichungstag der Anmeldung: 22.06.2022
(73) Patentinhaber: Evonik Operations GmbH, 45128 Essen (DE)
(72) Erfinder: Reuter, Ellen, 44799 Bochum (DE); Silber, Stefan, 47804 Krefeld (DE); Allef, Petra, 45128 Essen (DE); Roland, Katrin, 45257 Essen (DE); Cavaleiro, Pedro, 50858 Köln (DE); Mergenthaler, Jochen, 45219 Essen (DE); Jansen, Claudia, 51371 Leverkusen (DE); Blei, Marco, 45138 Essen (DE)
(74) Vertreter: Evonik Patent Association

(56) Entgegenhaltungen:
- WO-A1-2018/220577
- CN-A- 105 602 367
- US-A1- 2013 296 461
- US-A1- 2020 140 694

## Beschreibung

Die Erfindung betrifft die Verwendung von Rhamnolipiden und/oder Sophorolipiden in Beschichtungszusammensetzungen zur Erhaltung eines homogenen Farbbildes des trockenen Beschichtungsfilms und/oder zur Erhöhung der Arbeitssicherheit bei der Herstellung von Beschichtungszusammensetzungen.

Beschichtungen werden auf Oberflächen zu dekorativen Zwecken und/oder zu Schutzzwecken aufgebracht. Für viele Beschichtungsaufgaben werden Formulierungen eingesetzt, die Partikel wie Pigmente und/oder Füllstoffe enthalten.

Pigmente sind farbgebende Substanzen, die im Anwendungsmedium unlöslich dispergiert vorliegen. Anwendungsmedien sind beispielsweise Farben, Lacke, Pigmentpräparationen, Druckfarben oder auch organische Lösungsmittel und sonstige Zubereitungen, in denen das Pigment eingearbeitet wird. Üblicherweise werden die bei der Synthese entstandenen Rohpigmente vermahlen oder zerkleinert angewendet, z. B. werden sog. Trockenmahl- und Nassmahlverfahren eingesetzt. Partikel mit kleinem Durchmesser neigen jedoch besonders stark zur Agglomeration und müssen deshalb stabilisiert werden.

Die Stabilisierung der Pigmente ist in der Lackindustrie von großer Bedeutung, denn Pigmente bestimmen als wichtiger Formulierungsbestandteil das optische Erscheinungsbild und die physikalisch-chemischen Eigenschaften einer Beschichtung. Damit sie in der Beschichtung ihre Wirkung optimal entfalten können, müssen sie während des Dispergierprozesses gleichmäßig und feinteilig im Lack verteilt werden. Die Verteilung muss stabilisiert werden, damit dieser Zustand bei der Herstellung, der Lagerung, der Verarbeitung und der anschließenden Filmbildung erhalten bleibt. Eine Wiedervereinigung der Primärteilchen und Aggregate kann z.B. zu ungenügender Farbtiefe, Ausschwimmen der Pigmente und/oder schlecht reproduzierbaren Farbtönen führen.

Handelsübliche Lackformulierungen sind nur in einem Teil der Anwendungsfälle monopigmentiert, häufig wird es sich um Mischungen von zwei oder mehreren verschiedenen Pigmenten handeln. Auch in solchen Systemen sollten alle Pigmente gut benetzt und möglichst deflockuliert und gleichmäßig im gesamten Lackfilm verteilt sein. Wenn diese Mischung aber gestört wird, weil die Pigmente sich entmischen, kommt es zu Farbtonveränderungen im Lack. Dieser Defekt wird als "Ausschwimmen" bezeichnet.

Eine der Ursachen für das Entmischen der Pigmente sind Strömungserscheinungen im trocknenden Lackfilm. Während der Trocknungsphase eines Lackfilms muss Lösemittel aus den unteren Lackschichten an die Oberfläche transportiert werden, beim Verdunsten erhöht sich die Dichte des zurückbleibenden Materials und es kommt zum Absinken. Zusätzlich treten beim Verdunsten Abkühlungseffekte auf und die Oberflächenspannung verändert sich. All das führt zur Ausbildung von Wirbelströmungen, die sich in Form von mehr oder weniger gleichmäßigen sechseckigen Zellen (den sogenannten Bénard-Zellen) äußern können. Im Zentrum der Zellen steigt das Lackmaterial nach oben, verteilt sich dann über die Oberfläche und strömt an den Zellgrenzen wieder nach unten. Diese Zellströmungen sind schon seit langem - nicht nur im Lack - bekannt. In einem pigmentierten System nehmen auch die Pigmente an diesen Wirbelströmungen teil und solange die Beweglichkeit der verschiedenen Pigmente ähnlich ist, werden sie auch in ganz ähnlicher Weise in den Wirbeln transportiert und es kommt nicht zur Entmischung. Sind aber die Pigmentbeweglichkeiten deutlich unterschiedlich, so ist auch das Transportverhalten unterschiedlich und es kann zur Entmischung kommen.

Durch diesen Effekt weist der trockene Beschichtungsfilm zum Beispiel ein fleckiges Aussehen auf.

Der Rub-out Test inklusive Bestimmung der Farbtondifferenz (Delta E) der geriebenen zur ungeriebenen Fläche ist eine gängige Methode, um diese Ausschwimm-Effekte oder auch Flokkulations-Effekte zu prüfen, dies ist zum Beispiel in Groteklaes M, Rub-out-Effekt, RD-18-01991 (2005) in Böckler F., Dill B., Eisenbrand G., Faupel F., Fugmann B., Gamse T., Matissek R., Pohnert G., Rühling A., Schmidt S., Sprenger G., RÖMPP [Online], Stuttgart, Georg Thieme Verlag, [November 2021] https://roempp.thieme.de/lexicon/RD-18-01991 beschrieben. Diese Effekte haben einen negativen Einfluss auf das homogene Farbbild des trockenen Lackfilmes. Daneben erfolgt die Beurteilung dieser unerwünschten Effekte der getrockneten Lackfilme häufig visuell unter Normlichtbedingungen.

Anwendungsmedien, Beschichtungssysteme, Beschichtungszusammensetzungen, Lackformulierungen, Lackmedien werden im Rahmen der vorliegenden Erfindung als Synonyme verwendet.

Aus dem Stand der Technik sind z.B. polymere Netz- und Dispergiermittel bekannt, die zum einen pigmentaffine Gruppen wie Carboxyl-, Amino- oder Phenylfunktionalitäten und zum anderen im Medium lösliche Seitenketten enthalten. Die pigmentaffinen Gruppen sollen dabei idealerweise eine schnelle Orientierung an die Oberfläche der Pigmente sowie eine hohe Permanenz auf ihr besitzen. Die Seitenketten sorgen für die Kompatibilität mit dem Dispergier- bzw Lackmedium und für eine sterische Stabilisierung der dispergierten Phase.

In einigen Veröffentlichungen werden auch Biotenside, oder auch Bio-Surfactants genannt, als Dispergiermittel, als Entschäumer, als Netzmittel oder als Emulgator in Farben und Lacken erwähnt. Bekannte Vertreter dieser Bio-Surfactants sind Rhamnolipide und Sophorolipide. Diese Lipide werden heutzutage mit Wildtyp-Isolaten verschiedener Hefen, insbesondere mit Candida bombicola, hergestellt.

Die EP 3 006 505 beschreibt zum Beispiel eine Beschichtungszusammensetzung, die eine Dispersion bestehend aus einem Bindemittel, einem Biozid und einem Biotensid bestehend aus Rhamnolipiden und Sophorolipiden, enthält. Die EP 2 847 285 offenbart eine ähnliche Zusammensetzung, wobei das Isothiazolinon-Biozid konkret zugesetzt wird. Beide Schriften offenbaren die synergistische Biozid-Wirkung der Rhamnolipide und Sophorolipide mit einem Biozid.

Bei der Einarbeitung der feingemahlenen Partikeln in das Anwendungsmedium kann es zu einer Staubbelastung in der Produktionshalle kommen. In diesem Arbeitsschritt öffnen bekanntermaßen z.B. die Beschäftigten Säcke mit den Partikeln und schütten den Inhalt in ein offenes Behältnis, worin das Anwendungsmedium vorgelegt wurde. In großen Produktionsanlagen werden die in Silos aufbewahrten Partikel in das Anwendungsmedium zugegeben. Anschließend wird der Einrührvorgang gestartet. Es ist auch bekannt, den Einrührvorgang während der Zugabe der Partikel durchzuführen. Dabei wird - solange Pigmente als Pulver auf der Oberfläche des flüssigen Anwendungsmediums liegen - viel Staub aufgewirbelt. Feine Partikel können in die feinen Lungenbläschen vordringen und dort Krankheiten verursachen. Viele Unternehmen haben deswegen Absaugvorrichtungen installiert, um die Staubbelastung für die Beschäftigten zu minimieren. Zusätzlich müssen die Beschäftigten häufig Atemmasken tragen, was unter dem Atemschutz viel Hitzestau bedeutet.

Es wäre daher wünschenswert, Substanzen zu identifizieren, die die Staubbelastung bei der Herstellung von Beschichtungszusammensetzungen reduzieren. Auch wünschenswert wäre es, Substanzen in Beschichtungszusammensetzungen zu verwenden, um ein homogenes Farbbild der trockenen Beschichtungsfilme zu erreichen, ohne sonstige Eigenschaften zu beeinträchtigen, wie z. B. den Farbton oder den Farbort.

Überraschenderweise wurde nun gefunden, dass die Verwendung von Rhamnolipiden und/oder Sophorolipiden in Beschichtungszusammensetzungen zur Verbesserung der Benetzungsgeschwindigkeit von pulverförmigen Formulierungsbestandteilen in solchen Zusammensetzungen, was zu einer Verminderung der Staubbelastung während deren Einarbeitung und somit zur Erhöhung der Arbeitssicherheit bei der Herstellung von Beschichtungszusammensetzungen führt, geeignet ist, wobei die Erhaltung eines homogenen Farbbildes des trockenen Beschichtungsfilms gewährleistet ist bzw. verbessert wird.

Es konnte zudem festgestellt werden, dass der trockene Beschichtungsfilm durch die Verwendung von Rhamnolipiden und/oder Sophorolipiden ein verbessertes homogenes Farbbild im Hinblick auf den Farbtonunterschied, ermittelt anhand des Delta E, und der visuellen Bewertung, wie unten beschrieben, aufweist, im Vergleich zu einem herkömmlichen Beschichtungsfilm ohne Rhamnolipide und/oder Sophorolipide.

Ein weiterer Vorteil ist die Verkürzung der Einbringungsdauer der Partikel in das Anwendungsmedium, was eine Optimierung der Prozessdauer bedeutet.

Pulverförmige Formulierungsbestandteile, Feststoffe, Pigmente und Partikel werden als Synonyme verwendet.

Vorzugsweise handelt es sich bei den Beschichtungszusammensetzungen um Zubereitungen für verschiedene Anwendungsbereiche, die durch Auftragsverfahren wie z. B. Spritz-, Tauch-, Walz-, Gieß-, Rollen- oder Streichapplikation sowie verschiedene Druckverfahren auf den zu beschichtenden Untergrund aufgebracht werden.

Beispiele für Beschichtungsstoffe im Sinne der vorliegenden Erfindung sind Farben, Lacke, Druckfarben und sonstige Beschichtungsstoffe, wie lösemittelhaltige oder wässrige Lacke und lösemittelfreie Lacke, Pulverlacke, UV-härtbare Lacke, Low-Solids, Medium-Solids, High-Solids, Automobillacke, Holzlacke, Einbrennlacke, 2K-Lacke, Metallbeschichtungsstoffe, Tonerzusammensetzungen. Weitere Beispiele für Beschichtungsstoffe werden in "Bodo Müller, Ulrich Poth, Lackformulierung und Lackrezeptur, Lehrbuch für Ausbildung und Praxis, Vincentz Verlag, Hannover (2003)" und "P.G. Garrat, Strahlenhärtung, Vincent Verlag "Hannover (1996)" genannt.

Bevorzugt handelt es sich bei den Farben und Lacken um Innenwandfarben, Fassadenfarben, Bautenfarben, Fussbodenbeschichtungen, Holzlacke, Industrielacke, Lacke für Automotive OEM (Original Equipment Manufacturer) oder Refinish, Grundierungen, Füller, Basislacke sowie Decklacke.

Beispiele für Druckfarben und/oder Drucklacke im Sinne der vorliegenden Erfindung sind lösemittelbasierende oder wässrige Druckfarben, Flexodruckfarben, Tiefdruckfarben, Buchdruck- bzw. Hochdruckfarben, Offsetdruckfarben, Lithographie-Druckfarben, Druckfarben für den Verpackungsdruck, Siebdruckfarben, Drucktinten, wie Drucktinten für Tintenstrahldrucker, Ink Jet Tinte, Drucklacke, wie Überdrucklacke. Weitere Druckfarben- und/oder Drucklackformulierungen sind in "E.W. Flick, Printing Ink and Overprint Varnish Formulations - Recent Developments, Noyes Publications, Park Ridge NJ, (1990)" und nachfolgende Auflagen genannt.

Bevorzugt weisen die Beschichtungszusammensetzungen Feststoffe auf, ausgewählt aus den Gruppen der Füllstoffe, Pigmente, Farbstoffe, optischen Aufheller, keramischen Materialien, magnetischen Materialien.

Beispiele für Pigmente sind solche aus der Gruppe der anorganischen Pigmente, wie Ruße, Titandioxide, Zinkoxide, Preußischblau, Eisenoxide, Cadmiumsulfide, Chrompigmente, wie zum Beispiel Chromate, Molybdate und gemischte Chromate und Sulfate des Bleis, Zink, Barium, Calcium und deren Mischungen. Weitere Beispiele für anorganische Pigmente werden in dem Buch "H. Endriss, Aktuelle anorganische Bunt-Pigmente, Vincentz Verlag, Hannover (1997)" genannt.

Beispiele für organische Pigmente sind solche aus der Gruppe der Azo-, Diazo-, kondensierten Azo-, Naphtol-, Metallkomplex-, Thioindigo-, Indanthron-, Isoindanthron-, Anthanthron-, Anthrachinon-, Isodibenzanthron-, Triphendioxazin-, Chinacridon-, Perylen-, Diketopyrrolopyrrol und Phtalocyaninpigmente. Weitere Beispiele für organische Pigmente werden in dem Buch "W. Herbst, K.Hunger, Industrial Organic Pigments, VCH, Weinheim (1993)" genannt.

Beispiele für Füllstoffe sind solche aus den Gruppen Talk, Kaolin, Kieselsäuren, Baryte und Kalk; keramische Materialien, wie zum Beispiel Aluminiumoxide, Silikate, Zirkonoxide, Titanoxide, Bornitride, Siliziumnitride, Borcarbide, gemischte Silizium-Aluminiumnitride und Metall-Titanate; magnetische Materialien, wie zum Beispiel magnetische Oxide von Übergangsmetallen, wie Eisenoxide, Kobalt dotierte Eisenoxide und Ferrite; Metalle, wie zum Beispiel Eisen, Nickel, Kobalt und deren Legierungen.

Dem Fachmann ist bekannt, dass in derartigen Beschichtungszusammensetzungen weitere Inhaltsstoffe enthalten können. Als flüssiges Medium können sie organische Lösemittel (z. B. Acetate wie Butyl- oder Ethylacetat, Kohlenwasserstoffe wie Testbenzine verschiedener Siedebereiche, Alkohole, Ether, Glykole und Glycolether) und/oder Wasser enthalten, wie dies in Abhängigkeit von den verwendeten Bindemitteln als Stand der Technik bekannt ist.

Es können übliche Bindemittel eingesetzt werden. Vorzugsweise können Alkyd-, Acrylat-, Styrolacrylat-, Epoxy-, Polyvinylacetat-, Polyester- oder Polyurethanbindemitteln verwendet werden. Dabei ist jede Art der Aushärtung möglich, beispielsweise oxidativ trocknend, physikalisch trocknend, selbstvernetzend; UV- oder Elektronenstrahlhärtend oder vernetzend durch Einbrennen.

Es ist vorstellbar, dass die Beschichtungszusammensetzung weitere Zusatzstoffe enthalten, wie zum Beispiel bevorzugt Netzmittel, Dispergieradditive, Rheologieadditive, Verlaufshilfsmittel oder Entschäumer.

Dem Fachmann ist bekannt, dass die Herstellung von Beschichtungszusammensetzungen, die Partikel enthalten, zum Beispiel über die Verwendung von Pigmentkonzentraten, Farbpasten, Pigmentpasten oder flüssigen Zubereitungen der Partikeln erfolgen kann. Es ist allerdings auch möglich, ein Mahlgut herzustellen, um dieses zeitnah für die Herstellung von Beschichtungszusammensetzungen einzusetzen.

Daher ist Gegenstand der Erfindung vorzugsweise auch die Verwendung von Rhamnolipiden und/oder Sophorolipiden in Pigmentkonzentraten, Farbpasten, Pigmentpasten oder flüssigen Zubereitungen der Partikeln oder Mahlgütern.

Rhamnolipide und Sophorolipide sind Tenside, die mittels Fermentation hergestellt werden können.

Rhamnolipide setzen sich aus ein bis zwei Rhamnose-Einheiten und ein bis drei, meist β-Hydroxy-Fettsäuren zusammen. Die Fettsäuren können gesättigt oder ungesättigt sein.

Die Variation in der Kettenlänge und Menge (Kongener) der Fettsäureanteile ist in einigen Veröffentlichungen beschrieben (Howe et al., FEBS J. 2006; 273(22):5101-12; Abdel-Mawgoud et al., Appl Microbiol Biotechnol, 86, 2010; S. 1323-1336).

Miao et al., Journal of Surfactants and Detergents, 17 (6), 2014; 1069-1080, beschreibt die Synthese von di-Rhamnolipidethylester durch die Veresterung mit Ethanol sowie die Eignung der Ester als nichtionisches Tensid.

WO2001010447 und EP1889623 offenbaren die pharmazeutischen und kosmetischen Anwendungen von Rhamnolipiden und kurzkettigen Rhamnolipidestern (C1-C6; Methyl- bis Hexylester, linear oder verzweigt), insbesondere bei der Wundheilung.

Bevorzugt handelt es sich bei den Rhamnolipiden um Verbindungen gemäß der allgemeinen Formel (I) oder deren Salze 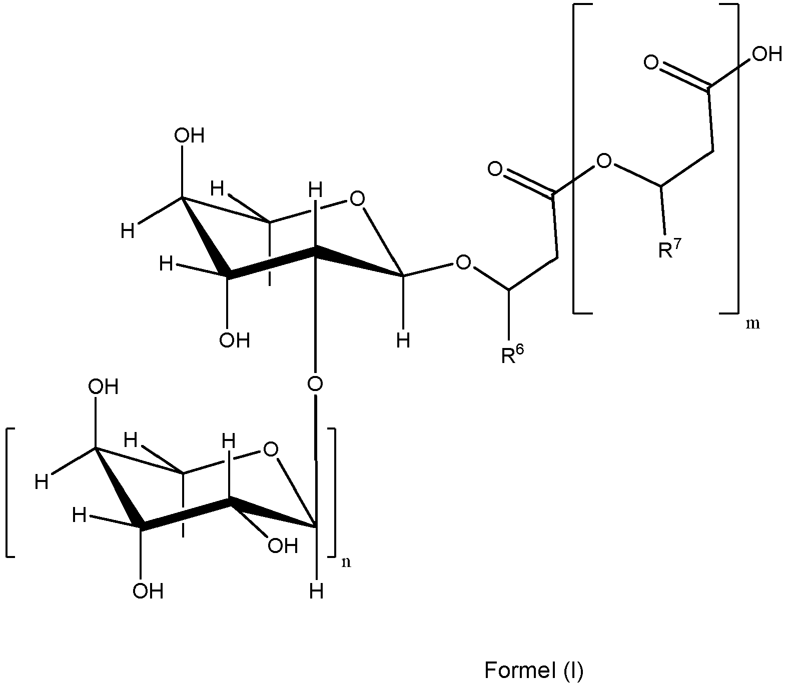 wobei
m = 2, 1 oder 0, insbesondere 1 oder 0,
n = 1 oder 0, insbesondere 1,
R⁶ und R⁷ = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH2)ₓ-CH3 mit x = 1 bis 23, bevorzugt 4 bis 12.

Bevorzugt wird eine Mischungszusammensetzung von Rhamnolipiden eingesetzt, die > 90% diRL enthält.

Bevorzugt wird eine Mischungszusammensetzung von Rhamnolipiden eingesetzt, die
51 Gew.-% bis 95 Gew.-% diRL-C10C10 und
0,5 Gew.-% bis 9 Gew.-% monoRL-C10C10, wobei sich die Gewichtsprozente auf die Summe aller enthaltenen Rhamnolipide beziehen, mit der Maßgabe, dass das Gewichtsverhältnis von di-Rhamnolipiden zu mono-Rhamnolipiden größer 91:9, bevorzugt größer 97:3, besonders bevorzugt größer 98:2 ist, aufweist.

Bevorzugt wird für erfindungsgemäße Verwendung eine Rhamnolipiden-Mischung enthaltend 0,5 Gew.-% bis 15 Gew.-% diRL-C10C12:1, wobei sich die Gewichtsprozente auf die Summe aller enthaltenen Rhamnolipide beziehen, verwendet.

Vorzugsweise handelt es bei den Sophorolipiden um Verbindungen gemäß der Formel (II) oder (IIa), 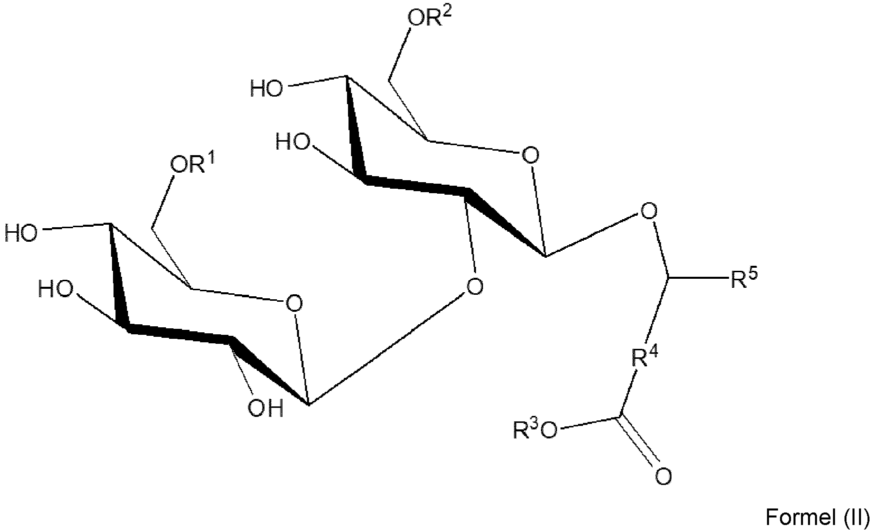 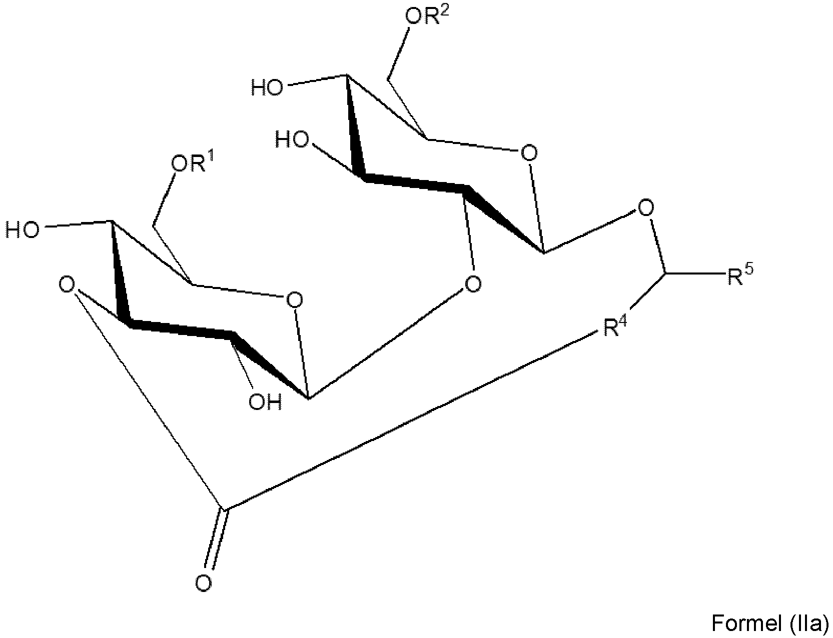 wobei
R¹ und R² unabhängig voneinander entweder H oder eine Acetylgruppe sind,
R³ H, eine Methyl-, Ethyl- oder Hexylgruppe ist,
R⁴ unabhängig voneinander eine gesättigte oder ungesättigte zweiwertige/zweibindige, verzweigte oder unverzweigte organische Gruppe ist,
R⁵ H oder eine Methylgruppe ist,
mit der Maßgabe, dass die Gesamtzahl der Kohlenstoffatome in den Gruppen R⁴ und R⁵ die Zahl 29 nicht überschreitet.

Rhamnolipide sind unter der Bezeichnung Natsurfact bei Fa. Stepan (Northfield, IL, USA) sowie RHEANCE^{®} One bei Evonik Operations GmbH erhältlich.

Sophorolipide sind unter der Bezeichnung HoneySurf bei Fa. Holiferm Limited (Manchester, UK) sowie REWOFERM^{®} SL ONE bei Evonik Operations GmbH erhältlich.

Bevorzugt werden Rhamnolipide und/oder Sophorolipide in einem Bereich von 0,01 Gew.-% bis 10,0 Gew.-% besonders bevorzugt 0,1 Gew.-% bis 5,0 Gew.-%, bezogen auf die Gesamtbeschichtungszusammensetzung eingesetzt.

Bevorzugt werden die Rhamnolipide und Sophorolipide gemäß den allgemeinen Formeln (I), (II) und (IIa) zur Beschleunigung der Benetzung von pulverförmigen Formulierungsbestandteilen in Beschichtungszusammensetzungen verwendet.

Bevorzugt werden die Rhamnolipide und Sophorolipide gemäß den allgemeinen Formeln (I), (II) und (IIa) zur Optimierung der Prozessführung bei der Herstellung von Beschichtungszusammensetzungen oder Pigmentkonzentraten, Farbpasten, Pigmentpasten oder Mahlgütern verwendet.

Nachstehend soll die Erfindung anhand von Ausführungsbeispielen näher erläutert werden.

### Methoden

### Rub-out Test (Delta E) und visuelle Bewertung

Für den "rub out Test" wurden die Beschichtungszusammensetzungen mit einem 150 µm Rakel von Leneta auf einem Prüfuntergrund bzw. Prüfsubstrat appliziert. Nach 5 Minuten Trockenzeit wurde der ,rub-out Test' durchgeführt, der darin besteht, den aufgezogenen Lack bei kreisförmigen Bewegungen mit einem Reibkörper wie z.B. einem behandschuhten Finger mit mittlerem Anpressdruck zu durchmischen. Dabei soll weder ein völliges Wegschieben der Beschichtung vom Untergrund noch ein Aufreißen des Lackfilms eintreten.

Die Farbwerte werden nach Trocknung der Lackfilme für 7 Tage bei Raumtemperatur auf der Fläche des "Rub-out" Tests sowie auf einer benachbarten, nicht dem "Rub-out" Test unterzogenen Fläche bestimmt. Aus beiden Werten wird das ΔE (Delta E) berechnet. Diese Differenz der Farborte ist ein Maß für die Güte der Pigmentstabilisierung.

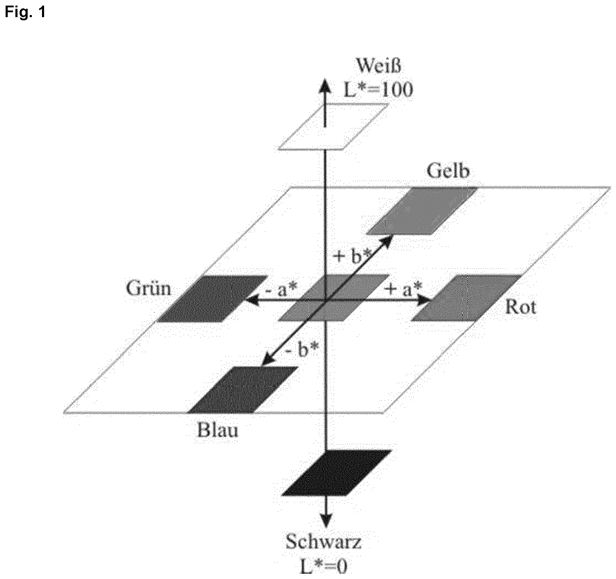

Die Farbwerte sowie der Delta E-Wert wurden mit einem Spektrometer Model SP 62 von X-Rite bestimmt. Der L*a*b*-Farbraum beschreibt dabei bekanntermaßen alle wahrnehmbaren Farben. Er nutzt einen dreidimensionalen Farbenraum, bei dem der Helligkeitswert L* senkrecht auf der Farbebene (a*,b*) steht. Das Farbmodell ist in der EN ISO 11664-4 "Colorimetry -- Part 4: CIE 1976 L*a*b* Colour space" genormt. Typischerweise werden vom Messgerät die Werte L*, a* und b* vermessen. Diese Werte ergeben dann einen Farbort in einem Koordinatensystem, wobei L* die Helligkeit angibt (0 = schwarz; 100 = weiß), a* den grün/rot-Wert (- grün/ + rot) und b* den blau/gelb-Wert (-blau/ + gelb). (siehe Fig. 1)

Die visuelle Bewertung der getrockneten Filme im Hinblick auf die Fleckigkeit sowie die Farbtonunterschiede der geriebenen Fläche im Vergleich zur ungeriebenen Fläche erfolgt in einer Farbabmusterungskabine mit D65 Daylight Beleuchtung mit folgender Bewertungsskala:
0 = homogen, keine Flecken, keine Farbunterschiede
1 = sehr schwache Flecken, sehr schwache Farbtonunterschiede
2 = schwache Flecken, schwache Farbtonunterschiede
3 = starke Flecken, starke Farbtonunterschiede
4 = sehr starke Flecken, sehr starke Farbtonunterschiede

### Beispiele

### 1. Herstellung der Pigmentkonzentrate

Es wurden zunächst Pigmentkonzentrate gemäß den Angaben aus den jeweiligen Tabellen 1.2 - 1.4 hergestellt. Zur Herstellung dieser Pigmentkonzentrate wurden zuerst die flüssigen Bestandteile eingewogen und danach die Pigmente zugegeben. Die Dispergierung erfolgte nach Zugabe von Mahlkörpern (Glaskugeln 2-3 mm Durchmesser, gleiches Volumen wie das Pigmentkonzentrat) eine (anorg. Pigmente) beziehungsweise zwei Stunden (organische Pigmente und Ruß) in einem Skandex-Rüttler (FAS 500 der Fa. Lau GmbH) mit Luftkühlung. Die Mahlkörper wurden abgesiebt. Das entstehende Filtrat ist das Pigmentkonzentrat.

Die Vergleichsbeispiele wurden analog mit Tego^{®} Dispers 755 hergestellt.

**Tabelle 1.1 Verwendete Materialien**

| **Handelsname** | **Beschreibung** | **Hersteller** |
|---|---|---|
| Wasser, deionisiert | | Evonik Operations GmbH |
| Bayferrox 130 M | Pigment | Lanxess AG |
| Heliogen Blue L7101F | Pigment | BASF AG |
| Special Black 4 | Pigment, Ruß | Orion Engineered Carbons GmbH |
| Tego^{®} Dispers 755 | Copolymer | Evonik Operations GmbH |
| Rewoferm^{®} SL One | Sophorolipid | Evonik Operations GmbH |
| RHEANCE^{®} One | Rhamnolipid | Evonik Operations GmbH |
| Tego^{®} Foamex 830 | Entschäumer | Evonik Operations GmbH |
| Tego^{®} Foamex 810 | Entschäumer | Evonik Operations GmbH |
| Aerosil^{®} 200 | Füllstoff | Evonik Operations GmbH |

**Tabelle 1.2: Pigmentkonzentrate mit Bayferrox 130 M**

| | **VBay** | **Bay1** | **Bay2** |
|---|---|---|---|
| Produkt | Menge in g | Menge in g | Menge in g |
| Wasser, deionisiert | 47,4 | 47,4 | 47,4 |
| Tego^{®} Dispers 755 | 30,0 | | |
| Rewoferm^{®} SL One | | 30,0 | |
| RHEANCE^{®} One | | | 30,0 |
| Tego^{®} Foamex 830 | 2,0 | 2,0 | 2,0 |
| Aerosil^{®} 200 | 0,6 | 0,6 | 0,6 |
| Bayferrox^{®} 130 M | 120,0 | 120,0 | 120,0 |

**Tabelle 1.3: Pigmentkonzentrate mit Heliogen Blue L7101F**

| | **VBlue** | **Blue1** | **Blue2** |
|---|---|---|---|
| Produkt | Menge in g | Menge in g | Menge in g |
| Wasser, deionisiert | 42,1 | 42,1 | 42,1 |
| Tego^{®} Dispers 755 | 21,9 | | |
| Rewoferm^{®} SL One | | 21,9 | |
| RHEANCE^{®} One | | | 21,9 |
| Tego^{®} Foamex 810 | 1,0 | 1,0 | 1,0 |
| Heliogen Blue L7101F | 35,0 | 35,0 | 35,0 |

**Tabelle 1.4: Pigmentkonzentrate mit Special Black 4**

| | **VBlack** | **Black1** | **Black2** |
|---|---|---|---|
| Produkt | Menge in g | Menge in g | Menge in g |
| Wasser, deionisiert | 42,8 | 42,8 | 42,8 |
| Additiv 1 | 31,2 | | |
| Additiv 2 | | 31,2 | |
| Additiv 3 | | | 31,2 |
| Tego^{®} Foamex 810 | 1,0 | 1,0 | 1,0 |
| Special Black 4 | 25,0 | 25,0 | 25,0 |

### 2. Bestimmung des Delta E-Wertes

Für die anwendungstechnische Prüfung wurden die jeweiligen Pigmentkonzentrate aus Beispiel 1 in eine wässrige Beschichtungszusammensetzung überführt:
0,73 g Pigmentkonzentrat und 19,27g eines Lacks der Marke ContiPur Satin der Fa Kluthe auf Polyurethanbasis wurden in einer 50 ml Kosmetikdose eingewogen und mit Hilfe eines Speed Mixers DAC 150 FVZ (Firma Hauschild) für 1 min bei 2.000 U/min homogenisiert.

Danach wurde jeweils ein "Rub Out Test" durchgeführt. Man erhält dabei folgende Ergebnisse:

**Tabelle 2:**

| | **L*** | **a*** | **b*** | **Rubout ΔE** |
|---|---|---|---|---|
| **VBay** | 66,58 | 19,30 | 6,60 | 1,08 |
| **Bay1** | 67,58 | 18,73 | 6,04 | 0,61 |
| **Bay2** | 67,48 | 18,04 | 4,57 | 0,85 |
| **VBlue** | 65,14 | -21,95 | -34,39 | 1,07 |
| **Blue1** | 68,09 | -21,67 | -31,85 | 1,04 |
| **Blue2** | 65,73 | -21,77 | -34,07 | 0,58 |
| **VBlack** | 49,05 | -0,80 | -3,35 | 1,01 |
| **Black1** | 49,19 | -0,83 | -3,63 | 0,74 |
| **Black2** | 49,59 | -0,81 | -3,67 | 0,68 |

Aufgrund der geringen Delta E-Werte weisen die durch die erfindungsgemäße Verwendung erhaltenen trockenen Farbfilme weniger Ausschwimm-Effekte auf als die Vergleichsbeispiele. Dies lässt die Schlussfolgerung zu, dass der trockene Farbfilm ein homogenes Erscheinungsbild bzw. Farbbild aufweist.

**Tabelle 2a: Visuelle Bewertung**

| | **Visuelle Bewertung** |
|---|---|
| **VBay** | 4 |
| **Bay1** | 2 |
| **Bay2** | 3 |
| **VBlue** | 4 |
| **Blue1** | 3 |
| **Blue2** | 2 |
| **VBlack** | 4 |
| **Black1** | 2 |
| **Black2** | 2 |

Die visuelle Bewertung ergibt, dass die durch die erfindungsgemäße Verwendung erhaltenen trockenen Farbfilme deutlich weniger Farbtonunterschiede sowie eine deutlich geringere Fleckigkeit aufweisen als die Vergleichsbeispiele.

### 3. Prüfung des Pigmentbenetzungsverhaltens

Zur Prüfung des Benetzungsverhaltens von Pigmenten wurden in eine 100ml Glas-Weithalsflasche jeweils 50g demin. Wasser eingefüllt. Dann wurde die entsprechende Menge der Rhamnolipide und Sophorolipide gemäß Tabelle 3.1 zugegeben und durch Umschwenken für 30 Sekunden homogenisiert zur Erhaltung eines Lipid-Wassergemisches. Danach wurden 0,1g des Pigmentes Heliogen Blau L7101F auf die Oberfläche die Lipid-Wassergemisches gegeben und die Benetzung sowie das Einsinkverhalten des Pigmentes beobachtet.

Für das Vergleichsbeispiel (VBenetzung) wurde das handelsübliche TEGO Dispers 755 W verwendet.

Man erhält dabei folgende Ergebnisse:

**Tabelle 3.1 Pigmentbenetzungsverhalten**

| | **VBenetzung** | **Benetzung1** | **Benetzung2** |
|---|---|---|---|
| | **A** | **B** | **C** |
| | Menge in g | Menge in g | Menge in g |
| Demineralisiertes Wasser | 50,0 | 50,0 | 50,0 |
| TEGO Dispers 755 W | 0,1 | | |
| Rewoferm SL ONE | | 0,1 | |
| RHEANCE^{®} One | | | 0,1 |
| Heliogen Blau L7101F | 0,1 | 0,1 | 0,1 |
| Zeit bis zur vollständigen Benetzung [min] | >>60:00 | 01:43 | 00:48 |

Es zeigte sich, dass die Pigmente aufgrund der erfindungsgemäßen Verwendung schneller benetzt werden können. Bei dem Vergleichsbeispiel dauerte der Benetzungsvorgang über 60 Minuten.

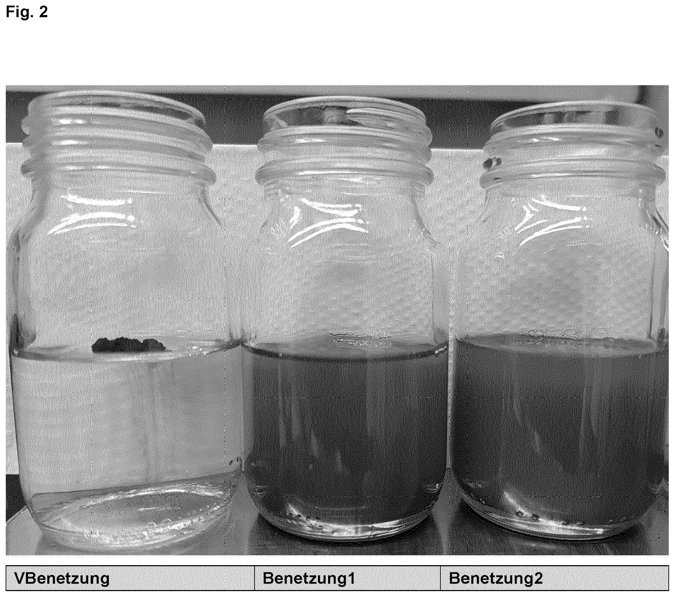

**Fig. 2** zeigt ein Foto der 100ml Glas-Weithalsflaschen nach 2 Minuten. Man erkennt deutlich, dass auf der Oberfläche des TEGO Dispers/Wassergemisches (VBenetzung) die Pigmente liegen. Während bei den erfindungsgemäßen Beispielen die Pigmente in dem Lipid-Wassergemisch benetzt und verteilt werden.

## Patentansprüche

1. Verwendung von Rhamnolipiden und/oder Sophorolipiden in Beschichtungszusammensetzungen zur Verbesserung der Benetzungsgeschwindigkeit von pulverförmigen Formulierungsbestandteilen in solchen Zusammensetzungen, was zu einer Verminderung der Staubbelastung während deren Einarbeitung führt, und somit zur Erhöhung der Arbeitssicherheit bei der Herstellung von Beschichtungszusammensetzungen sowie zur Erhaltung eines verbesserten homogenen Farbbildes des trockenen Beschichtungsfilms im Hinblick auf den Farbtonunterschied, ermittelt anhand des Delta E, und der visuellen Bewertung.

2. Verwendung nach Anspruch 1, **dadurch gekennzeichnet, dass** es sich bei den Beschichtungszusammensetzungen um Farben und Lacke handelt, ausgewählt aus Innenwandfarben, Fassadenfarben, Bautenfarben, Fussbodenbeschichtungen, Holzlacken, Industrielacken, Autoserien- oder Reparaturlacken, Grundierungen, Füllern, Basislacken sowie Decklacken.

3. Verwendung nach Anspruch 1, **dadurch gekennzeichnet, dass** die Verwendung in Pigmentkonzentraten, Farbpasten, Pigmentpasten oder Mahlgütern erfolgt.

4. Verwendung nach einem der vorgenannten Ansprüche, **dadurch gekennzeichnet, dass** die Beschichtungszusammensetzungen Feststoffe ausgewählt aus Füllstoffen Pigmenten, Farbstoffen, optischen Aufhellern, keramischen Materialien, magnetischen Materialien aufweisen.

5. Verwendung nach einem der vorgenannten Ansprüche, **dadurch gekennzeichnet, dass** die Beschichtungszusammensetzungen weitere Zusatzstoffe enthalten, bevorzugt Netzmittel, Dispergieradditive, Rheologieadditive, Verlaufshilfsmittel oder Entschäumer.

6. Verwendung nach einem der vorgenannten Ansprüche, **dadurch gekennzeichnet, dass** es sich bei den Rhamnolipiden um Verbindungen gemäß der allgemeinen Formel (I) oder dessen Salz 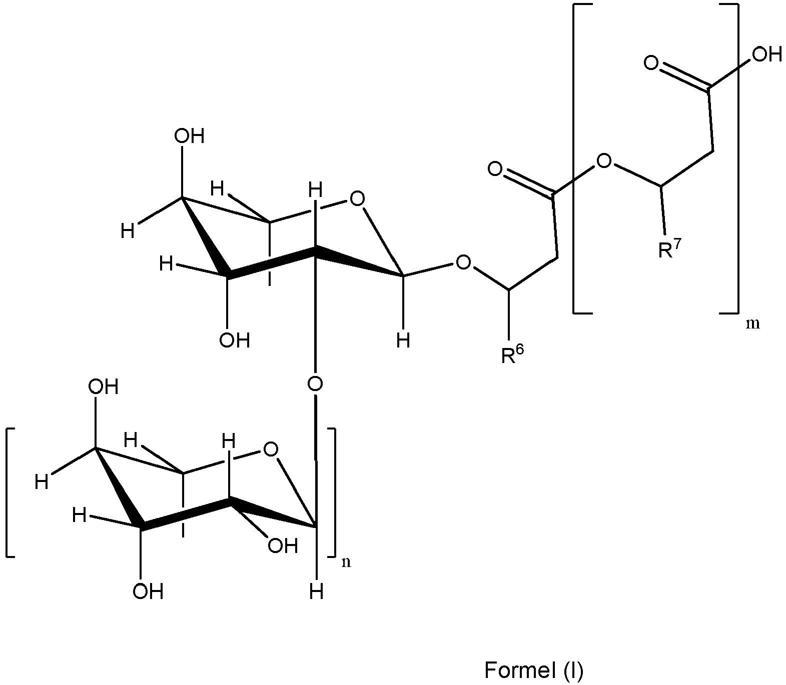 wobei
m = 2, 1 oder 0, insbesondere 1 oder 0,
n = 1 oder 0, insbesondere 1,
R⁶ und R⁷ = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₓ-CH₃ mit x = 1 bis 23, bevorzugt 4 bis 12, handelt.

7. Verwendung nach einem der vorgenannten Ansprüche, **dadurch gekennzeichnet, dass** es sich bei den Rhamnolipiden um eine Mischungszusammensetzung mit > 90% diRL handelt.

8. Verwendung nach einem der vorgenannten Ansprüche, **dadurch gekennzeichnet, dass** es sich bei den Rhamnolipiden um Mischungszusammensetzung enthaltend Rhamnolipide handelt, **dadurch gekennzeichnet, dass** die Mischungszusammensetzung
51 Gew.-% bis 95 Gew.-% diRL-C10C10 und
0,5 Gew.-% bis 9 Gew.-% monoRL-C10C10
enthält, wobei sich die Gewichtsprozente auf die Summe aller enthaltenen Rhamnolipide beziehen, mit der Maßgabe, dass das Gewichtsverhältnis von di-Rhamnolipiden zu mono-Rhamnolipiden größer 91:9, bevorzugt größer 97:3, besonders bevorzugt größer 98:2 ist.

9. Verwendung nach einem der vorgenannten Ansprüche, **dadurch gekennzeichnet, dass** es sich bei den Rhamnolipiden um Mischungszusammensetzung enthaltend Rhamnolipide handelt, **dadurch gekennzeichnet, dass** die Mischungszusammensetzung 0,5 Gew.-% bis 15 Gew.-% diRL-C10C12:1 enthält, wobei sich die Gewichtsprozente auf die Summe aller enthaltenen Rhamnolipide beziehen.

10. Verwendung nach einem der vorgenannten Ansprüche, **dadurch gekennzeichnet, dass** es sich bei den Sophorolipiden um Verbindungen gemäß der Formel (II) oder (IIa) handelt, 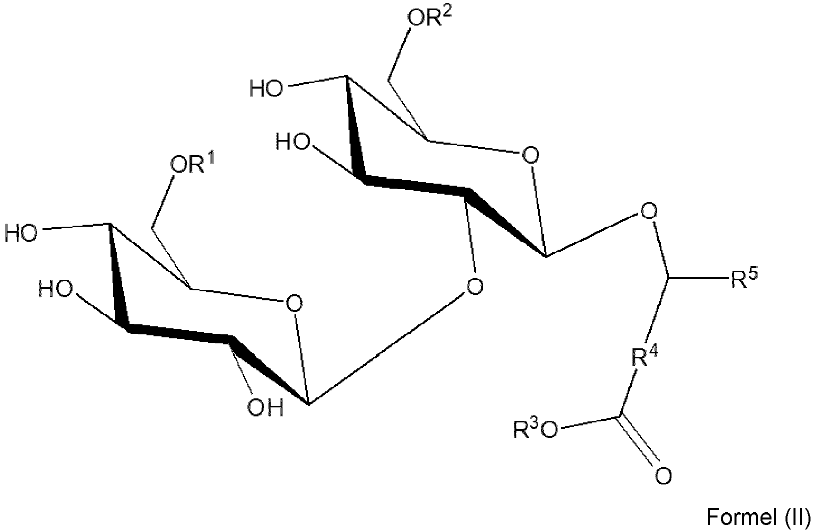 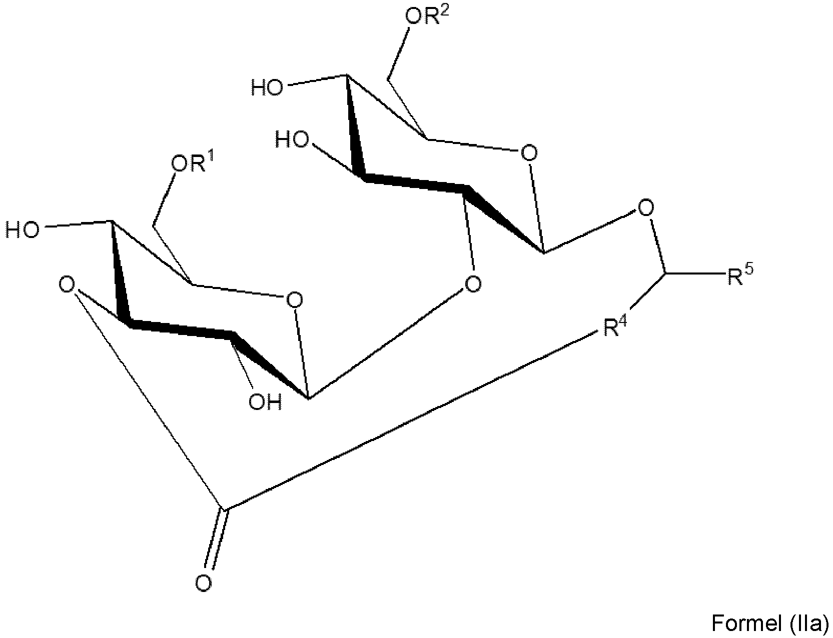 wobei
R¹ und R² unabhängig voneinander entweder H oder eine Acetylgruppe sind,
R³ H, eine Methyl-, Ethyl- oder Hexylgruppe ist,
R⁴ unabhängig voneinander eine gesättigte oder ungesättigte zweiwertige/zweibindige, verzweigte oder unverzweigte organische Gruppe ist,
R⁵ H oder eine Methylgruppe ist,
mit der Maßgabe, dass die Gesamtzahl der Kohlenstoffatome in den Gruppen R⁴ und R⁵ die Zahl 29 nicht überschreitet.

11. Verwendung nach einem der vorgenannten Ansprüche, **dadurch gekennzeichnet, dass** Rhamnolipide und/oder Sophorolipide in einem Bereich von 0,01 Gew.-% bis 10,0 Gew.-% besonders bevorzugt 0,1 Gew.-% bis 5,0 Gew.-%, bezogen auf die gesamte Beschichtungszusammensetzung eingesetzt werden.

## Claims

1. Use of rhamnolipids and/or sophorolipids in coating compositions for improvement of the speed of wetting of pulverulent formulation constituents in such compositions, which leads to a reduction in dust nuisance during incorporation thereof, and hence to the increase in occupational safety in the production of coating compositions and to the obtaining of improved homogeneous colour appearance of the dry paint film with regard to difference in hue, ascertained from the delta E, and visual assessment.

2. Use according to Claim 1, **characterized in that** the coating compositions are paints and coating materials selected from interior wall paints, exterior paints, architectural paints, floor coatings, wood paints, industrial paints, automotive OEM or refinishing paints, primers, primer-surfacers, basecoats and topcoats.

3. Use according to Claim 1, **characterized in that** the use takes place in pigment concentrates, colour pastes, pigment pastes or millbases.

4. Use according to any of the preceding claims, **characterized in that** the coating compositions include solids selected from fillers, pigments, dyes, optical brighteners, ceramic materials, magnetic materials.

5. Use according to any of the preceding claims, **characterized in that** the coating compositions comprise further additives, preferably wetting agents, dispersing additives, rheology additives, levelling aids or defoamers.

6. Use according to any of the preceding claims, **characterized in that** the rhamnolipids are compounds of the general formula (I) or a salt thereof 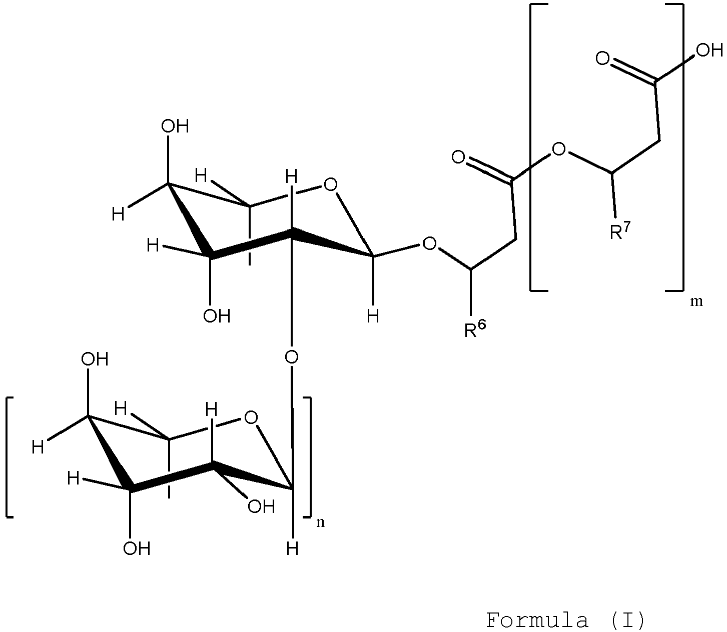 where
m = 2, 1 or 0, especially 1 or 0,
n = 1 or 0, especially 1,
R⁶ and R⁷ = independently an identical or different organic radical having 2 to 24, preferably 5 to 13, carbon atoms, especially optionally branched, optionally substituted, especially hydroxy-substituted, optionally unsaturated, especially optionally mono-, di- or triunsaturated, alkyl radical, preferably those selected from the group consisting of pentenyl, heptenyl, nonenyl, undecenyl and tridecenyl and (CH₂)ₓ-CH₃ with x = 1 to 23, preferably 4 to 12.

7. Use according to any of the preceding claims, **characterized in that** the rhamnolipids are a mixed composition with > 90% diRL.

8. Use according to any of the preceding claims, **characterized in that** the rhamnolipids are a mixed composition comprising rhamnolipids, **characterized in that** the mixed composition contains
51% by weight to 95% by weight of diRL-C10C10 and
0.5% by weight to 9% by weight of monoRL-C10C10,
where the percentages by weight are based on the sum total of all rhamnolipids present, with the proviso that the weight ratio of di-rhamnolipids to mono-rhamnolipids is greater than 91:9, preferably greater than 97:3, more preferably greater than 98:2.

9. Use according to any of the preceding claims, **characterized in that** the rhamnolipids are a mixed composition comprising rhamnolipids, **characterized in that** the mixed composition contains 0.5% by weight to 15% by weight of diRL-C10C12:1, where the percentages by weight are based on the sum total of all rhamnolipids present.

10. Use according to any of the preceding claims, **characterized in that** the sophorolipids are compounds of the formula (II) or (IIa) 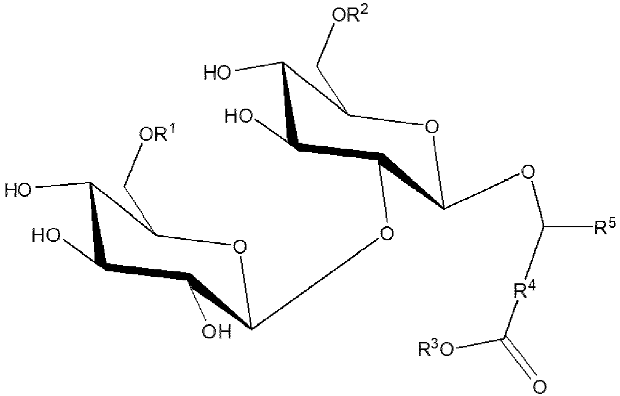 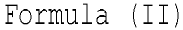 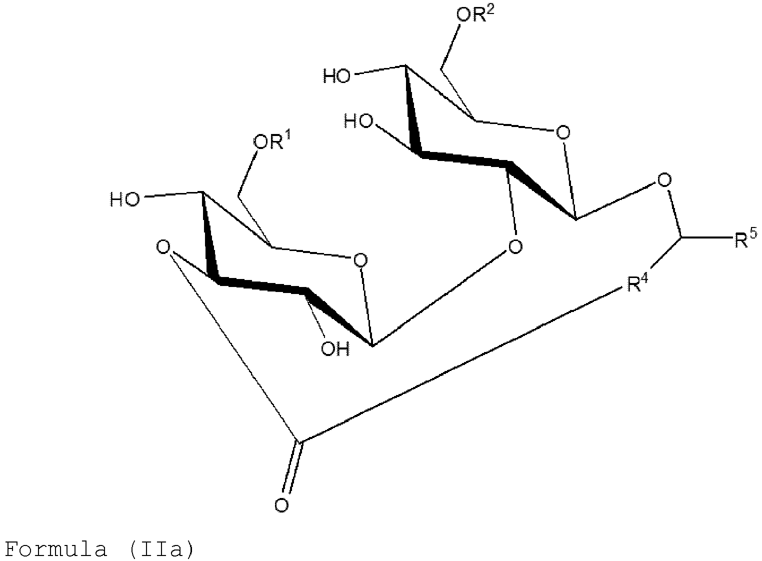 where
R¹ and R² are independently either H or an acetyl group,
R³ is H, a methyl, ethyl or hexyl group,
R⁴ is independently a saturated or unsaturated divalent branched or unbranched organic group,
R⁵ is H or a methyl group,
with the proviso that the total number of the carbon atoms in the groups R⁴ and R⁵ does not exceed the number 29.

11. Use according to any of the preceding claims, **characterized in that** rhamnolipids and/or sophorolipids are used within a range from 0.01% by weight to 10.0% by weight, more preferably 0.1% by weight to 5.0% by weight, based on the overall coating composition.

## Revendications

1. Utilisation de rhamnolipides et/ou sophorolipides dans des compositions de revêtement pour améliorer la vitesse de mouillage de constituants pulvérulents de formulation dans ces compositions, ce qui entraîne une diminution de la charge en poussière pendant leur incorporation et donc une augmentation de la sécurité de travail lors de la préparation de compostions de revêtement, ainsi que pour obtenir un aspect de couleur homogène amélioré du film de revêtement sec eu égard à la différence de nuance, déterminée à l'aide du Delta E et de l'évaluation visuelle.

2. Utilisation selon la revendication 1, **caractérisée en ce qu'**il s'agit, pour les compositions de revêtement, de peintures et de laques choisies parmi les peintures murales intérieures, les peintures de façade, les peintures architecturales, les revêtements de sol, les laques pour bois, les laques industrielles, les laques de série pour voitures ou les laques de réparation, les apprêts, les agents de remplissage, les laques de base et les laques de recouvrement.

3. Utilisation selon la revendication 1, **caractérisée en ce que** l'utilisation a lieu dans les concentrats pigmentaires, les pâtes colorées, les pâtes pigmentaires ou les matières broyées.

4. Utilisation selon l'une quelconque des revendications susmentionnées, **caractérisée en ce que** les compositions de revêtement présentent des solides choisis parmi les charges, les pigments, les colorants, les azurants optiques, les matériaux céramiques, les matériaux magnétiques.

5. Utilisation selon l'une quelconque des revendications susmentionnées, **caractérisée en ce que** les compositions de revêtement contiennent d'autres additifs, de préférence des agents mouillants, des additifs dispersants, des additifs rhéologiques, des adjuvants d'étalement ou des antimousses.

6. Utilisation selon l'une quelconque des revendications susmentionnées, **caractérisée en ce qu'**il s'agit, pour les rhamnolipides, de composés selon la formule générale (I) ou son sel 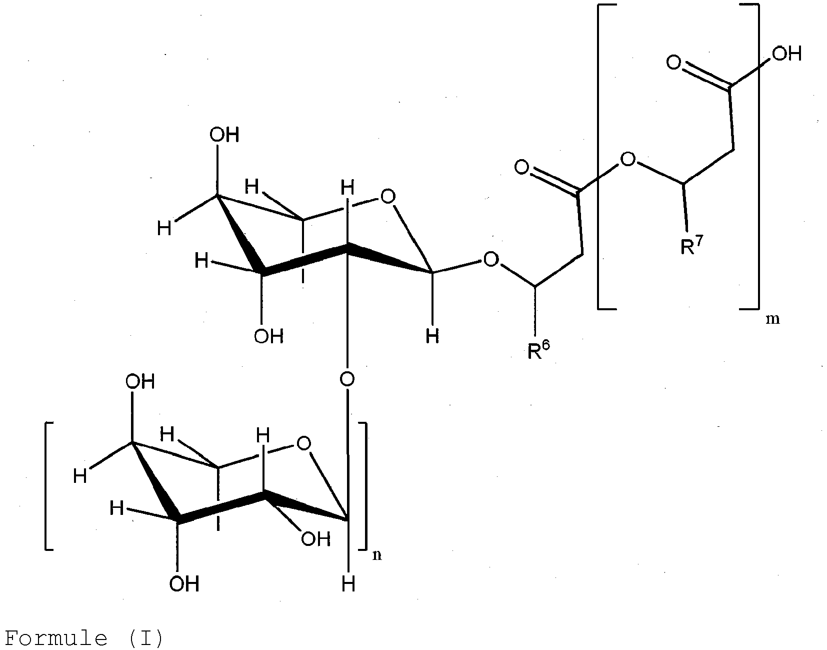 dans laquelle
m = 2, 1 ou 0, en particulier 1 ou 0,
n = 1 ou 0, en particulier 1,
R⁶ et R⁷ = indépendamment l'un de l'autre, des radicaux organiques identiques ou différents comprenant 2 à 24, de préférence 5 à 13, atomes de carbone, en particulier des radicaux alkyle le cas échéant ramifiés, le cas échéant substitués, en particulier substitués par hydroxy, le cas échéant insaturés, en particulier le cas échéant mono-insaturés, di-insaturés ou tri-insaturés, de préférence des radicaux choisis dans le groupe constitué par pentényle, heptényle, nonényle, undécényle et tridécényle et (CH₂)ₓ-CH₃, où x = 1 à 23, de préférence 4 à 12.

7. Utilisation selon l'une quelconque des revendications susmentionnées, **caractérisée en ce qu'**il s'agit, pour les rhamnolipides, d'une composition mélangée présentant > 90% de diRL.

8. Utilisation selon l'une quelconque des revendications susmentionnées, **caractérisée en ce qu'**il s'agit, pour les rhamnolipides, d'une composition mélangée contenant des rhamnolipides, **caractérisée en ce que** la composition mélangée contient
51% en poids à 95% en poids de diRL-C10C10 et
0,5% en poids à 9% en poids de monoRL-C10C10,
les pour cent en poids se rapportant à la somme de tous les rhamnolipides contenus, à condition que le rapport pondéral de di-rhamnolipides à mono-rhamnolipides soit supérieur à 91:9, de préférence supérieur à 97:3, de manière particulièrement préférée supérieur à 98:2.

9. Utilisation selon l'une quelconque des revendications susmentionnées, **caractérisée en ce qu'**il s'agit, pour les rhamnolipides, d'une composition mélangée contenant des rhamnolipides, **caractérisée en ce que** la composition mélangée contient 0,5 en poids à 15% en poids de diRL-C10C12:1, les pour cent en poids se rapportant à la somme de tous les rhamnolipides contenus.

10. Utilisation selon l'une quelconque des revendications susmentionnées, **caractérisée en ce qu'**il s'agit, pour les sophorolipides, de composés selon la formule (II) ou (IIa), 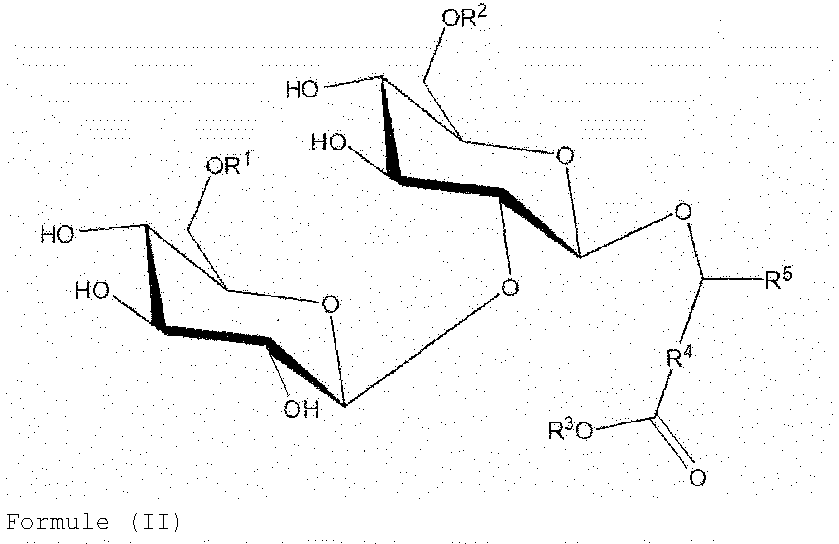 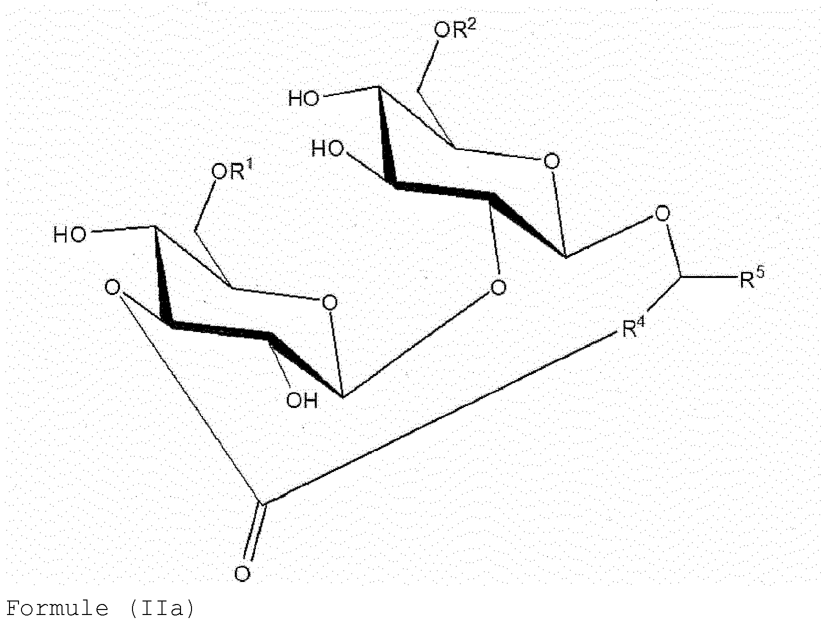 dans lesquelles
R¹ et R² représentent, indépendamment l'un de l'autre, soit H, soit un groupe acétyle,
R³ représente H, un groupe méthyle, éthyle ou hexyle,
R⁴ représente, indépendamment, un groupe organique saturé ou insaturé, divalent, ramifié ou non ramifié,
R⁵ représente H ou un groupe méthyle,
à condition que le nombre total d'atomes de carbone dans les groupes R⁴ et R⁵ ne dépasse pas le nombre 29.

11. Utilisation selon l'une quelconque des revendications susmentionnées, **caractérisée en ce que** les rhamnolipides et/ou les sophorolipides sont utilisés dans une plage de 0,01% en poids à 10,0% en poids, de manière particulièrement préférée de 0,1% en poids à 5,0% en poids, par rapport à la totalité de la composition de revêtement.
